# EUROPEAN PATENT SPECIFICATION

(11) **EP 1 502 596 B1**
(45) Date of publication and mention of the grant of the patent: **26.11.2008**
(21) Application number: 03720580.4
(22) Date of filing: 16.04.2003
(51) Int. Cl.: A61K 36/61, A61K 36/48, A61P 3/10

(54) **HERBAL PRODUCT TO BE ADMINISTERED TO DIABETIC INDIVIDUALS AND THE PRODUCTION METHOD THEREOF**
PFLANZLICHES PRODUKT ZUR VERABREICHUNG AN DIABETIKER UND SEIN HERSTELLUNGSVERFAHREN
PRODUIT A BASE DE PLANTES A ADMINISTRER A DES PERSONNES DIABETIQUES ET SON PROCEDE D'OBTENTION

(30) Priority: 23.04.2002 ES 200200943
(43) Date of publication of application: 02.02.2005
(73) Proprietor: Agreda Navajas, Juan Carlos, 01005 Vitoria (ES); Martin Pinto, Fidel, 01005 Vitoria (ES)
(72) Inventor: BELO MALUENDAS, Efren, William, E-01005 Vitoria (ES); AGREDA NAVAJAS, Juan, Carlos, E-01005 Vitoria (ES); MARTIN PINTO, Fidel, E-01005 Vitoria (ES)
(74) Representative: Hart-Davis, Jason
(86) International application number: PCT/ES2003/000177
(87) International publication number: WO 2003/090768

(56) References cited:
- WO-A1-03/011311
- FR-A- 2 465 484
- GB-A- 1 281 526
- US-A- 5 900 240
- CACERES T. ET AL.: 'O efeito dos chas de pata de vaca (bauhinia forticata) e jambolao (Syzygium jambolanum) na reducao da glicemia em ratos diabeticos', [Online] 06 December 2002, page 1, XP002904595 Retrieved from the Internet: <URL:http://search.cometsystems.com/search. php?qyr=www.asbran.org.br/docs/PO181.htm> [retrieved on 2003-04-15]
- LEMUS I. ET AL.: 'Hypoglycaemic activity of four plants used in chilean popular medicine' PHYTOTHERAPY RESEARCH vol. 13, no. 2, 1999, pages 91 - 94, XP002904920
- KOHLI K.R. ET AL.: 'Eugenia jambolana: A plant drug with potential antidiabetic property (a Review)' J. SCI. RES. P1. MED. vol. 6, no. 1-4, 1985, pages 21 - 28, XP000940820
- LEMUS I. ET AL.: 'Action hypoglycemiante de l'extrait de Bauhinia candicans B' PLANTES MEDICINALES & PHYTOTHERAPIE vol. 20, no. 1, 1986, pages 8 - 17, XP009023915
- GROVER J.K. ET AL.: 'Anti-hyperglycemic effet of Eugenia jambolana and tinospora cordifolia in experimental diabetes and their effects on key metabolic enzymes involved in carbohydrate metabolism' JOURNAL OF ETHNOPHARMACOLOGY vol. 73, no. 3, 2000, pages 461 - 470, XP002904902
- ABD-EL-WAHAB S.M. ET AL.: 'Flavonoid constituents in the different organs of selected Bauhinia species and their effect on blood glucose' HERBA HUNGARICA vol. 26, no. 1, 1987, pages 27 - 39, XP009023910

## Description

The object of the present invention is a herbal product developed and appropriate for the administration to diabetic people and more specifically to people who suffer from diabetes type II, not dependent from insulin.

The formulation of this herbal product is made up of the mixture of two plants, one of them belonging to the Myrtaceae and the other to the family of the leguminosae - caesalpinoideae. Specifically, the first of them is a Syzygium and the second a Bauhinia.

This product has a hypoglycaemic activity. Its mimetic activity with human insulin allows to reduce the glycose levels in patients with diabetes type II; in an unedited way in pharmacology of herbal formulations.

On the other hand and as they are products obtained from the mixture of two natural plants, the collateral effects on the patients are low or almost nonexistent, which supposes a great advantage as compared to the traditional chemical formulae.

According to the present invention a herbal product is tried to obtain, for its administration to diabetic patients, and more specifically to those suffering from type II, not insulin dependent diabetes.

### FORMULATION

The qualitative formulation of this product is the one made up of the mixture of two plants, one of them belonging to the Myrtaceae and the other to the family of the leguminosae - caesalpinoideae.

Specifically, the plant belonging to the Myrtaceae is a Syzygium and more specifically in a preferable but not limitative way the species Syzygium cumini or Syzygium jambolanum as selected varieties.

The plant of the leguminosae - caesalpinoideae family is a Bauhinia and specifically in a preferable but not limitative way of the species Bauhinia spp.

The quantitative formulae is determined by a percentage by weight included between 50 % and 95 % of the Syzygium and a percentage by weight included between 5 % and 50 % of Bauhinia.

Within these percentages by weight satisfactory results have been reached with a percentage by weight of 85 % of Syzygium and a percentage by weight of 15 % of Bauhinia.

Particularly samples have been prepared of the product in which the weight of the plants was 450 mg. In this case, the amount of Syzygium is 382,5 mg and the amount of Bauhinia is 67,5 mg.

The presentation of the product can be any of the ones allowed by the pharmacopean techniques but preferably it shall be presented in capsules.

### PRODUCTION PROCESS

The process is started with the gathering or harvesting of the plants, after which the Syzygium seeds are cleaned as well as the Bauhinia leaves.

Both the seeds of Syzygium and the leaves of Bauhinia are pulverized to 60 mesh and afterwards dried till reaching 9 % humidity.

After a quality control process, the grinding phase is started, in which the seeds and the leaves are ground, after which they are sterilized.

In the following phase both ground plants are mixed till reaching a perfect homogenization, after which they are capsulized after passing the corresponding quality controls. Finally the finished product is packed.

### DOSAGE

As a possible administration dose, satisfactory results have been reached with the following dosage:
* A first attacking dose, according to which 4 capsules of 450 mg should be taken, each of them daily for a period of one week.
* A maintenance dose of two capsules of 450 mg, each of them daily during the remaining 51 weeks, to complete, between the attacking dose and the maintenance one a period of one year.

It is convenient that the capsule ingestion is carried out twice a day; so that during the attacking dose two capsules shall be taken before breakfast in the morning and two other ones before dinner.

A capsule shall be taken before breakfast and another one before dinner for the maintenance dose.

## Claims

1. Herbal product for administration to diabetic people suffering from diabetes type II, not insulin dependent, **characterized in that** it is made up of the mixture of two plants, one of which belongs to the Myrtaceae family and the other to the leguminosae - caesalpinoideae family,
wherein the plant belonging to the Myrtaceae family is a plant of the Syzygium species, and the plant belonging to the leguminosae - caesalpinoideae family is a Bauhinia.

2. Herbal product for administration to diabetic people according to claim 1, **characterized in that** the plant of the Myrtaceae family is a Syzygium cumini or Syzygium jambolanum, while the plant belonging to the leguminosae - caesalpinoideae family is a Bauhinia spp.

3. Herbal product for administration to diabetic people according to claim 1 or claim 2, **characterized in that** the percentage by weight of the Syzygium lies between 50 % and 95 %, while the percentage by weight of the Bauhinia lies between 50 % and 5 %.

4. Herbal product for administration to diabetic people according to the claim 3, **characterized in that** the percentage by weight of the Syzygium is 85 % while the percentage by weight of the Bauhinia is 15 %.

5. Herbal product for administration to diabetic people according to any of claims 1 to 4, **characterized in that** the presentation of the product is in capsules containing 450 mg as the combined weight of both plants.

6. Herbal product for administration to diabetic people according to claim 5, **characterized in that** 382,5 mg by weight correspond to the Syzygium cumini and 67,5 mg by weight correspond to the Bauhinia spp.

7. Process to obtain a herbal product for administration to diabetic people, **characterized in that** the process comprises the following steps:
* the harvesting of the plants and the separation of the seed corresponding to the Syzygium and the leaves of the Bauhinia;
* the cleaning by pulverizing of the Syzygium seeds and of the Bauhinia leaves;
* the subsequent drying until 9 % humidity is reached;
* the grinding of the Syzygium seeds and the Bauhinia leaves; their sterilization and subsequent homogenization by mixing;
* incorporation of the homogenized ground plants into capsules.

8. Use of a plant of the Syzygium species and a Bauhinia in the manufacture of a herbal combination product for the treatment of diabetes.

## Patentansprüche

1. Pflanzliches Produkt zur Verabreichung an Diabetiker, die an Diabetes Typ-II leiden, dem nicht-insulinabhängigen Diabetes, **dadurch gekennzeichnet, daß** es aus der Mischung von zwei Pflanzen besteht, wobei eine Pflanze zu der Familie der Myrtaceae und die andere zu der Leguminosae-Caesalpinoideae-Familie gehört,
wobei die Pflanze, die zur Familie der Myrtaceae gehört, eine Pflanze der Gattung Syzygium ist und die Pflanze, die zur Familie der Leguminosae-Caesalpinoideae gehört, eine Bauhinie ist.

2. Pflanzliches Produkt zur Verabreichung an Diabetiker nach Anspruch 1, **dadurch gekennzeichnet, daß** die Pflanze aus der Familie der Myrtaceae eine Syzygium cumini oder Syzygium jambolaneum ist und die Pflanze, die zur Familie der Leguminosae-Caesalpinoideae gehört, eine Bauhinie spp. ist.

3. Pflanzliches Produkt zur Verabreichung an Diabetiker nach Anspruch 1 oder Anspruch 2, **dadurch gekennzeichnet, daß** der prozentuale Gewichtsanteil von Syzygium zwischen 50 % und 95 % liegt, während hingegen der prozentuale Gewichtsanteil von der Bauhinie zwischen 50 % und 5 % liegt.

4. Pflanzliches Produkt zur Verabreichung an Diabetiker nach Anspruch 3, **dadurch gekennzeichnet, daß** der prozentuale Gewichtsanteil von Syzygium 85 % beträgt, während der prozentuale Gewichtsanteil von der Bauhinie 15 % beträgt.

5. Pflanzliches Produkt zur Verabreichung an Diabetiker nach einem der Ansprüche 1 bis 4, **dadurch gekennzeichnet, daß** die Darreichung von dem Produkt in Kapseln erfolgt, die 450 mg von dem zusammengefaßten Gewicht beider Pflanzen enthalten.

6. Pflanzliches Produkt zur Verabreichung an Diabetiker nach Anspruch 5, **dadurch gekennzeichnet, daß** ein Gewicht von 382,5 mg Syzygium cumini und ein Gewicht von 67,5 mg Bauhinia spp. entspricht.

7. Verfahren zum Erhalt eines pflanzlichen Produktes zur Verabreichung an Diabetiker, **dadurch gekenntzeichnet, daß** das Verfahren die folgenden Schritte umfaßt:
* Ernten der Pflanzen und das Abtrennen von den Samen, die zu dem Syzygium gehören und von den Blättern der Bauhinie;
* Einigen von den Syzygium-Samen und den Bauhinienblättern durch Pulverisieren;
* anschließendes Trocknen der Pflanzen, bis eine Feuchtigkeit von 9 % erreicht ist;
* Zerreiben von den Syzygium-Samen und den Bauhinienblättern; ihre Sterilisation und die nachfolgende Homogenisierung durch Mischen;
* Einarbeiten der homogenisierten, zerriebenen Pflanzen in Kapseln.

8. Verwendung von einer Pflanze der Gattung Syzygium und einer Bauhinie in der Herstellung von einem pflanzlichen Kombinationsprodukt für die Behandlung von Diabetes.

## Revendications

1. Produit végétal pour administration à des diabétiques souffrant de diabète de type II, non insulinodépendant, **caractérisé en ce qu'**il est constitué du mélange de deux plantes, dont l'une appartient à la famille des Myrtaceae et l'autre à la famille des leguminosae-caesalpinoïdeae,
où la plante appartenant à la famille des Myrtaceae est une plante de l'espèce Syzygium et la plante appartenant à la famille des leguminosae-caesalpinoïdeae est une Bauhinia.

2. Produit végétal pour administration à des diabétiques selon la revendication 1, **caractérisé en ce que** la plante de la famille des Myrtaceae est Syzygium cumini ou Syzygium jambolanum, alors que la plante appartenant à la famille des leguminosae-caesalpinoïdeae est une Bauhinia spp.

3. Produit végétal pour administration à des diabétiques selon la revendication 1 ou la revendication 2, **caractérisé en ce que** le pourcentage en poids de Syzygium se situe entre 50 et 95 %, alors que le pourcentage en poids de Bauhinia se situe entre 50 % et 5 %.

4. Produit végétal pour administration à des diabétiques selon la revendication 3, **caractérisé en ce que** le pourcentage en poids de Syzygium est de 85 %, alors que le pourcentage en poids de Bauhinia est de 15 %.

5. Produit végétal pour administration à des diabétiques selon l'une quelconque des revendications 1 à 4, **caractérisé en ce que** la présentation du produit se fait en capsules contenant 450 mg représentant le poids combiné des deux plantes

6. Produit végétal pour administration à des diabétiques selon la revendication 5, **caractérisé en ce que** 382,5 mg en poids correspondent à Syzygium cumini et 67,5 mg en poids correspondent à Bauhinia spp.

7. Procédé pour obtenir un produit végétal pour administration à des diabétiques, **caractérisé en ce que** le procécé comprend les étapes suivants :
* récolte des plantes et séparation des gratines correspondent à Syzygium et des feuilles correspondant à Bauhinia ;
* nettoyage par pulvérisation des gratines de Syzygium et des feuilles de Bauhinia ;
* séchage subséquent jusqu'à atteinte de 9 % d'humilité
* broyage des graines de Syzygium et des feuilles de Bauhinia ;
* stérilisation de celles-ci et homogénéisation subséquente par mélange ;
* incorporation des plantes broyées homogénéisées dans des capsules.

8. Utilisation d'une plante de l'espèce Syzygium et d'une Bauhinia dans la préparation d'un produit combiné végétal destiné au traitement du diabète.
